# EUROPEAN PATENT SPECIFICATION

(11) **EP 3 350 599 B1**
(45) Date of publication and mention of the grant of the patent: **02.09.2020**
(21) Application number: 16767265.8
(22) Date of filing: 19.09.2016
(51) Int. Cl.: G01N 33/573, G01N 33/68

(54) **IN VITRO METHOD AND KIT TO DIAGNOSE SKELETAL DISORDERS**
IN VITRO VERFAHREN UND KIT ZUR DIAGNOSE VON SKELETTKRANKHEITEN
MÉTHODE IN VITRO ET KIT POUR LE DIAGNOSTIC DES MALADIES DU SQUELETTE

(30) Priority: 18.09.2015 IT UB20153737
(43) Date of publication of application: 25.07.2018
(73) Proprietor: Università degli Studi di Roma "La Sapienza", 00185 Roma (IT)
(72) Inventor: LENZI, Andrea, 00185 Roma (RM) (IT); MIGLIACCIO, Silvia, 00185 Roma (RM) (IT)
(74) Representative: Capasso, Olga
(86) International application number: PCT/EP2016/072107
(87) International publication number: WO 2017/046410

(56) References cited:
- K. PANIGRAHI ET AL.: "Characteristics of a Two-Site Immunoradiometric Assay for Human Skeletal Alkaline Phosphatase in Serum", CLINICAL CHEMISTRY (AMERICAN CHEMICAL SOCIETY), vol. 40, no. 5, 1994, pages 822-828, XP055263080, Winston-Salem NC USA cited in the application
- F. SARAÇ AND F. SAYGILI: "Causes of High Bone Alkaline Phosphatase", BIOTECHNOLOGY & BIOTECHNOLOGICAL EQUIPMENT, vol. 21, no. 2, 1 January 2007 (2007-01-01), pages 194-197, XP055263076, Taylor & Francis Group,Milton Park UK ISSN: 1310-2818, DOI: 10.1080/13102818.2007.10817444 cited in the application

## Description

### FIELD OF THE INVENTION

The present invention falls within the field of diagnosis of skeletal disorders.

In particular, the invention relates to a method for the diagnosis and/or for the prediction of the risk of developing skeletal disorders comprising the selective detection and/or quantification of the human bone-specific isoform of alkaline phosphatase (BAP) using polyclonal antibodies directed against said bone-specific isoform of the alkaline phosphatase enzyme.

### PRIOR ART

Bone remodelling process in adult life consists in a highly dynamic process characterised by a delicate balance between the production of new bone tissue by osteoblasts and the resorption of aged or damaged bone by osteoclasts. An imbalance between these two processes leads to an alteration in bone homeostasis with an impoverishment of the skeletal tissue, which leads to pathologies that are often disabling¹. In particular, the most frequent pathology of skeletal tissue resulting from an alteration in the remodelling process is osteoporosis, which the World Health Organization has defined as a "systemic skeletal disease characterized by a reduction in bone strength that increases the risk of developing both spontaneous fractures and fractures induced by minimal traumas" ². To date, the diagnosis of osteoporosis has been based on an evaluation, by DXA (dual X-ray absorptiometry), of bone mineral density (BMD) in the lumbar vertebrae and proximal femur. The diagnosis is based on a T-score which indicates by how many "standard deviations" the BMD value observed in a subject is below the average BMD in a control population made up of healthy subjects of the same sex and young adults (at the age in which a peak in bone mass is reached). A T-score greater than -1 is considered normal, a T-score between -1 and -2.5 defines "osteopenia", and a T-score value lower than -2.5 leads to a diagnosis of osteoporosis³.

However, various limitations in the use of BMD values as a diagnostic and prognostic parameter of bone fragility have been highlighted. For example, a slight reduction in bone mineral density will not be detected, although it may represent a risk factor for fractures⁴. Moreover, the pharmacological treatment of osteoporosis induces slow, modest changes in the BMD, hence the effectiveness of the treatment in a subject cannot be rapidly monitored⁵.

Consequently, recently the use of molecular markers of bone remodelling (BTMs - bone turnover markers) has proven to be increasingly useful, as they have been shown to be sensitive for assessing risk of fracture and for monitoring the response to pharmacological treatments^{4-6,8-9}.

Moreover, BTMs can be evaluated by means of non-invasive methods (blood and/or urine sampling) that are thus easy to perform, in particular in laboratory testing. There are limitations, however, in the use of BTM assays, e.g. biological variability and the heterogeneity of analysis methods⁸.

Thus, there is still the need to provide a simple, economical, broad-spectrum method to evaluate BTMs in patients who require a relatively constant monitoring of their skeletal condition.

The Human Alkaline Phosphatase (AP) is an almost ubiquitous, enzyme¹³. There are at least four distinct but related genes coding for APs: intestinal, placental, placental-like, and liver/bone/kidney (tissue non-specific). The first three are located together on chromosome 2, while the tissue non-specific form is located on chromosome 1. The product of this gene is a membrane bound glycosylated enzyme of 524 aa, that is not expressed in any particular tissue and is, therefore, referred to as the tissue-nonspecific form of the enzyme.

The protein sequence has the following sequence (NCBI accession AAB59378.1, SEQ ID No. 3):

A specific bone-type alkaline phosphatase (BAP) mRNA was also identified from human periodontal ligament cells¹¹

Below is reported the partial coding sequence found in NCBI (accession number AH007388.2, SEQ ID No. 1):

Encoding for a protein whose sequence is not yet completely known (NCBI accession number AAD14985.2, SEQ ID No. 2:
>gi|4572574|gbAAD14985.2|1683459_1 bone-type alkaline phosphatase, partial [Homo sapiens] RARYPGSVLPRACAWTDPRQCSAQDWN IS

Alterations in serum BAP levels can be a characteristic of various skeletal disorders. Apart from hypophosphatasia, where the BAP levels, together with those of the other alkaline phosphatases (APs), are reduced, most diseases affecting bone homeostasis are characterized by a considerable increase in serum BAP levels and, for instance, increased serum BAP levels are also found in Paget's disease.

Serum AP, deriving from the post-translational cleavage by phospholipase of the tetrameric form, is made up of different dimeric isoforms originating from various tissues, such as: liver, bone, intestine, spleen, kidneys and placenta. In adults with normal liver function, about 50% of the total activity of AP derives from the liver and 50% from bones. The BAP has the advantage of presenting no variation between genders and not influenced by circadian rhythm, and it is easy to detect in the absence of specific clinical conditions such as gestation and liver disease¹². A diagnostic assay for bone pathologies should therefore be able to distinguish the two isoforms, so as to detect exclusively variations in the bone isoenzyme.

Moreover, in healthy adult subjects both liver and bone isoforms of AP are quantitatively present in the same percentage and the monoclonal antibodies show a 10-20% cross-reactivity between the two isoforms. In addition, other bone formation markers are used for monitoring skeletal modelling, In particular, Osteocalcin (OCN) is an osteoblast-specific secreted protein and a major non-collagenous protein in the extracellular matrix (1, 5). Interestingly uncarboxylated OCN can act as a pro-hormone, can increase β-cell proliferation, insulin secretion, insulin sensitivity, and adiponectin expression. Thus, evaluation of serum OCN levels can lead to evaluate osteoblasts activity. Conventional methods for determining the quantity of bone alkaline phosphatase mainly include: enzyme-linked immunosorbent assay (ELISA), high-performance liquid chromatography (HPLC), liquid chromatography tandem mass spectrometry (LC-MS/MS) and other techniques. Some of these techniques, like HPLC, cannot be exploited for analysis in loco or to analyze a large number of samples because of the highly sophisticated instruments, complexity of the process and the high level of skill required of operators. Other techniques are less accurate, time-consuming, or costly per se.

The patent application WO8907949 relates to monoclonal antibodies (mAbs) able to distinguish bone, liver and kidney ALP isozymes. These monoclonal antibodies are useful for quantitative analysis of these isozymes by methods known in the art.

Saraç F¹⁴ reports two patients with elevated bone specific alkaline phosphatase.

Panigrahi et al.¹⁵ describes a two-site immunoradiometric assay including two murine monoclonal antibodies directed against different antigenic sites on BAP for quantifying BAP in human serum.

Thus, there is still the need to provide a method, in particular an immunoassay, for quantifying the levels of human bone alkaline phosphatase in biological fluids. In particular there is the need for a method that is highly sensitive and specific in order to diagnose subjects at risk for or affected by skeletal disorders.

### SUMMARY OF THE INVENTION

The present authors have developed a method for determining the levels of human bone alkaline phosphatase (BAP) in a biological sample. The method enables both a high specificity and increased sensitivity to be achieved, using a polyclonal antibody directed against the bone tissue-specific isoform.

The method may also further comprises determining the levels of osteocalcin (OCN).

The invention therefore provides an *in vitro* method for the diagnosis of a skeletal disorder in a biological sample of a subject comprising the following steps:
a) contact and incubation of the biological sample with an anti-human bone alkaline phosphatase (BAP) polyclonal antibody thereby forming a BAP-antibody complex, if BAP is present;
b) separation of the biological sample from the BAP-antibody complex;
c) selective detection of BAP bound to the polyclonal antibody and/or quantifying the amount of BAP bound to the polyclonal antibody using detecting means for the antibody;
d) comparison of the result obtained in c) to a control result, wherein the biological sample is selected from the group consisting of: serum, plasma, saliva , urine, a supernatant of osteoblast cells, preferably wherein the osteoblast cells are *in vitro* cultivated and/or stimulated with subject's serum and
   wherein an amount of BAP greater than the amount of the control sample indicates that the subject is affected by a skeletal disorder selected from the group consisting of: osteoporosis, osteosarcoma, Paget's disease, osteodystrophy, osteopetrosis,
   or wherein an amount of BAP lower than the amount of the control sample indicates that the subject is affected by a skeletal disturb is hypophosphatasia.

Preferably the anti-human bone alkaline phosphatase (BAP) polyclonal antibody is immobilized to a solid support.

Preferably an amount of BAP greater or lower than the amount of the control sample indicates that the subject is affected by a skeletal disorder or is at risk of developing a skeletal disorder. Preferably the anti-human BAP polyclonal antibody is an IgG. Preferably the anti-human BAP polyclonal antibody is produced in sheep.

In a preferred embodiment the biological sample is selected from the group consisting of: serum, plasma, saliva, urine, a supernatant of osteoblast cells.

In a preferred embodiment the osteoblast cells are *in vitro* cultivated and/or stimulated with the subject's serum.

Still preferably the amount of BAP is greater than the amount of the control sample and the skeletal disorder is selected from the group consisting of: osteoporosis, osteosarcoma, Paget's disease, osteodystrophy, osteopetrosis.

Still preferably the amount of BAP is lower than the amount of the control sample and the skeletal disturb is hypophosphatasia.

More preferably the amount of a further skeletal disorder marker is detected and/or measured. Preferably the further skeletal disorder marker is osteocalcin.

The present invention also provides the use of a kit comprising:
an anti-BAP polyclonal antibody for carrying out the method as above defined. Preferably, said kit further comprises means to detect and/or quantify a BAP-antibody complex.

Preferably the method is an immunoassay. Still preferably the method detects and/or quantifies the human bone alkaline phosphatase (BAP) in the biological sample.

Yet preferably the kit further comprises a solid support wherein the antibody is immobilized. Preferably the solid support is a microtitration plate. Preferably the anti-BAP polyclonal antibody is an IgG. Still preferably the anti-human BAP polyclonal antibody is produced in sheep.

The means for detecting and/or quantifying the antigen-antibody complex are preferably means for detecting and/or quantifying the catalytic activity of ALPs such as secondary antibodies conjugated to enzymes, luminescent substrates. These means are known in the art.

The kit according to the invention can further comprise typical auxiliary components, such as buffers, carriers, dyes, etc. and/or instructions for use.

The kit can further comprise control means for comparing the increase in the amount of BAP with an appropriate control value. The control value can be obtained, for example, with reference to known standards, either from a normal subject or a normal population.

In the present invention when the subject is affected by or at risk of developing osteoporosis, osteosarcoma, Paget's disease, osteodystrophy and osteopetrosis, an increase in BAP is observed when compared to a healthy subject or to a subject not affected by a skeletal disorder.

In the present invention when the subject is affected by or at risk of developing hypophosphatasia a decrease in BAP is observed when compared to a healthy subject or to a subject not affected by a skeletal disorder.

In the present invention, the "control result" can be the result obtained for a sample isolated from a healthy subject or from a patient affected by another disorder than skeletal disorder.

In the case of a method for monitoring the progression of the skeletal disorder, the control result could be the result of a sample isolated from the same subject at various points in time prior to the start-up of the therapy, at various points in time during the therapy, etc.

In the case of a method for monitoring the efficacy of a therapy, the control sample can be a sample taken from the same subject prior to the start-up of the therapy or taken at various times during the therapy.

By "monitoring the efficacy" it is meant monitoring the pharmacological profile of an agent.

In the case of a method for screening a treatment of skeletal disorders, the control sample can be a sample taken from an untreated subject or a subject treated with a substance to be tested or a subject treated with a reference treatment. Reference treatment for a specific skeletal disorder is known to the skilled in the art.

By "screening" it is meant assessing whether an agent has a biological or pharmacological activity on a skeletal disorder.

In the present invention, the expression "detect" or "detection" refers to any use of any method of observation, assessment or quantification of the signals indicative of the antibody's presence in a sample or the absolute or relative amount of said antibody in a sample, for example by chemiluminescence, fluorimetry, spectrophotometry, etc. In the present invention, the expression "quantify" or "quantification" can be understood as measuring the amount or concentration or level of BAP or of the respective antibody, preferably with a semi-quantitative or quantitative method.

The term "amount", as used in the description refers to but is not limited to the absolute or relative amount of proteins, and any other value or parameter associated with the latter or which can derive therefrom. Such values or parameters comprise signal intensity values obtained either for physical or chemical properties of the protein, obtained by direct measurement, for example, intensity values in an immunoassay, mass spectroscopy or nuclear magnetic resonance. Moreover, these values or parameters include the ones obtained by indirect measurement.

In the present invention "skeletal disorders" include but is not limited to osteoporosis, osteosarcoma, Paget's disease, osteodystrophy and osteopetrosis.

Osteocalcin (OCN) is also known as bone gamma-carboxyglutamic acid-containing protein (BGLAP), is a noncollagenous protein found in bone and dentin. Because it has gla domains, its synthesis is vitamin K dependent. In humans, the osteocalcin is encoded by the *BGLAP* gene.^{[} The invention further relates to the use of the kit as defined above to carry out the method as defined above.

The major advantage of the present method compared to current methods and commercial kits for evaluating BAP in serum, is to take vantage of catalytic activity of BAP itself to detect the amount of the protein, without using a secondary antibody. The assay provides for the quantitative measurement of BAP, using a capture antibody anti-BAP to coat the wells of a polystyrene assay plate and exploiting the catalytic activity of the same protein bypassing therefore the use of a secondary antibody conjugated with an enzyme to catalyze the chromogenic reaction, as in common ELISA assays (sandwich). Alkaline phosphatase (ALP) activity is evaluated according to its ability to hydrolyze p-nitrophenyl phosphate (pNPP). Alkaline phosphatase (ALP) activity is proportional to the amount of p-nitrophenol (pNP) produced of yellow color that is measured using a spectrophotometer at a wavelength of 405-450 nm (maximum absorbance levels). The measurement of the product of the enzymatic reaction will be directly proportional to the concentration of BAP present in the test sample.

In this manner it is possible to avoid the use of two antibodies in the kit's assembly: the primary recognition antibody (used in normal sandwich ELISA procedures) and the secondary antibody tied to the signal detection enzyme; this means saving both time and money.

A further advantage of the kit and method of the invention is the possibility to increase the number of markers evaluated in a single plate.

The present invention method and kit present a sensitivity that is comparable or superior to that of kits already present on the market. Further with the present invention kit and method, BAP determinations are reproducible in different laboratories. In addition the present method and kit allow:
i) a reduction in preparation cost, by optimizing or excluding some reagents;
ii) a reduction in the number of antibodies to be used, by exploiting the intrinsic catalytic activity of the enzyme BAP;
iii) specificity for human bone alkaline phosphatase;
iv) a reduction of time needed to carry out the enzymatic assay (total of 5 hours);
v) reproducibility for the production of the kit;
vi) possibility to evaluate possible other bone markers within the same kit.

In particular, the present method may be an ELISA method, as it can be carried out in any clinical diagnostic centre, by any technical operator, without the need to purchase costly and complex instruments. In general, the ELISA technique is an assay used to detect the presence of markers of interest (almost always of a proteic nature) in biological fluid samples (e.g. serum, plasma and urine). ELISA exploits the ability of antibodies to specifically recognize a given antigen. In an ELISA assay, an unknown amount of antigen binds to a surface by binding to a specific antibody (primary antibody) or via a direct bond. A second specific antibody can then be used to increase the specificity of the assay (second primary antibody). The second primary antibody, or a so-called "secondary antibody" (which binds the second primary antibody), is linked to an enzyme capable of metabolizing a substrate which determines a detectable signal, for example, by means of a colorimetric assay.

The present invention will be described through non-limiting examples, making reference to the following figures:
**Fig. 1****. Example of a calibration curve**
**Fig. 2****. Example data**
**Fig. 3****. OCN calibration curve**

### DETAILED DESCRIPTION OF THE INVENTION

### Materials and methods

### BAP assay

Plates with 96 wells (442404 Nunc-Immuno Microplate 96-well Maxisorp, Thermo Scientific) characterized by a strong affinity for proteins (approx. 400 ng IgG/cm2) were coated with an anti-BAP polyclonal IgG antibody (produced in sheep) specific for bone alkaline phosphatase (Abcam Ab68716). The antigen used for antibody development is full length native protein from native human bone (purified).

The antibody was diluted in phosphate buffered saline (PBS - KH₂PO₄ 14,525 g, Na₂HPO₄^{×} 2H₂O 76g, NaCl 48g, H₂O to 10 litres, pH 7.5) at a concentration of 0.5 µg/ml and 70 µl was aliquoted into each well. The plate was dried in an oven at 37°C for 18 hours.

A standard curve was constructed using purified bone alkaline phosphatase from calf intestinal mucosa (AP; Santa Cruz sc-3716 - CAS9001-78-9). The protein is supplied by the manufacturer in a buffer: 50% glycerol, 5mM phosphate, 1mM MgCl₂ and 0.1 mM ZnCl₂ at a concentration of 10 mg/ml.

The BAP was further diluted in PBS buffer at pH 7.5 until the 6 points of the calibration curve were obtained: C [0] (0 ng/ml), C[1] (3.125 ng/ml), C [2] (6.25 ng/ml), C[3] (12.5 ng/ml), C[4] (25 ng/ml); C[5] (50 ng/ml), C[6] (100 ng/ml). 150 µl of each dilution was aliquoted (3 wells for each point on the curve). After 4 hours of incubation at 18-25°C on a tilt table, the wells were washed twice in PBS buffer with the addition of 0.05% Tween-20 and once in the same buffer without Tween-20 (200 µl per well). 150 µl of p-Nitrophenyl phosphate substrate (pNPP, Sigma - P7998) was aliquoted into each well and the plate was incubated on a tilt table in the dark at room temperature for 30 - 60 minutes.

The absorbance values were read with a spectrophotometer (Microplate Reader, BioRad-iMARK) at a wavelength of 415 nm.

The assay was also realized using (Ostease®BAP, IDS Ltd., UK, CODE:AC-20F1).

### Cell Culture and ELISA assay

Saos-2 osteoblastic cells, a human osteosarcoma osteoblast- like cells line (HTB-85; American Type Culture Collection), were cultured in Dulbecco's modified Eagle's medium (DMEM) without Phenol Red, supplemented with 2 mM L-glutamine, 100 U/mL penicillin/streptomycin, 10% fetal bovine serum (FBS), at 37°C and 5% CO₂ in a humidified incubator. Cells were incubated with the sera of subjects before and after physical activity 1) obese (SAOS-TO); obese after for 4 months (SAOS-T4); obese after 6 months (SAOS-T6) of physical activity and nutritional protocol intervention. For patients sera treatments, cells were grown up to 70% confluence, starved overnight in a medium serum-free, and then incubated 48 hrs with medium supplemented with 20% patients sera pools of the same group time All buffers, media, and reagents were purchased from Euroclone (Milan, Italy).

At the end of incubation, media were removed and centrifuged at 250 g for 12 minutes, and the supernatants were saved for the ELISA assay.

### Osteocalcin (OCN) assay

The 96-well plates (Nunc-Immuno 442404 Microplate 96-well Maxisorp, Thermo Scientific) which possess a strong affinity for proteins (∼400 ng IgG/cm²) were coated with polyclonal IgG Anti-OCN developed in rabbit (Abcam - Ab1857) that specifically recognizes human OCN. The antibody was diluted in PBS phosphate buffered saline (14.525 g KH2PO4, Na2HPO4 × 2H2O 76g, 48g NaCl, H2O at 10 liters, pH 7.2) at a concentration of 10 micrograms/ml and 50 microliters were aliquoted into each well. The plate was then incubated at 4°C for 18 hours. At the end of the incubation period, the wells were unfilled and emptied and the "blocking" step, was performed by incubating the wells with 50 microliters of BSA (0.1% in PBS) for 1 hour and 30 minutes at room temperature (RT).

The antigen used (1-22 aa peptide of human OCN - Abcam, Ab42234) was diluted in PBS with 0.1% BSA and different concentrations were tested (2, 10, 50 and 100 ng/ml). Fifty microliters of antigen dilution were aliquoted into each well and the plate incubated at RT for 3 hours. After thoroughly emptied wells, three washes were performed with 0.01% TBS-Tween 20 pH 7.0 (300 microliters/well). The antibody of "detection" (Abcam, Ab13418) was diluted in PBS to a concentration of 10 micrograms/ml, 100 microliters of each dilution was aliquoted into the appropriate wells and incubated for 1 hour at RT. After 2 washes in 0.01% TBS-Tween 20 pH 9.0, a goat anti-mouse IgG (H+L) secondary antibody (JIR laboratory 115-055-003) was diluted in TBS buffer at a concentration of 1 mg/ml and 50 microliters aliquoted into each well. After 1 hour incubation at RT, secondary antibody was removed and 3 washes in TBS pH 9.0 (300 µl / well) were performed. Then, 100 microliters of para-Nitrophenylphosphate substrate (pNPP, Sigma - P7998) were added in each well and the plate incubated 30-60 minutes on a tilting platform in the dark at RT. The absorbance values were evaluated at the spectrophotometer (Microplate Reader, BioRad-Imark) at a wavelength of 415 nm.

### Data Analysis

GraphPad Prism 5 software was used for the data analysis and graphic construction of the straight line from the interpolation of data.

### RESULTS

### BAP analysis

### Calibration

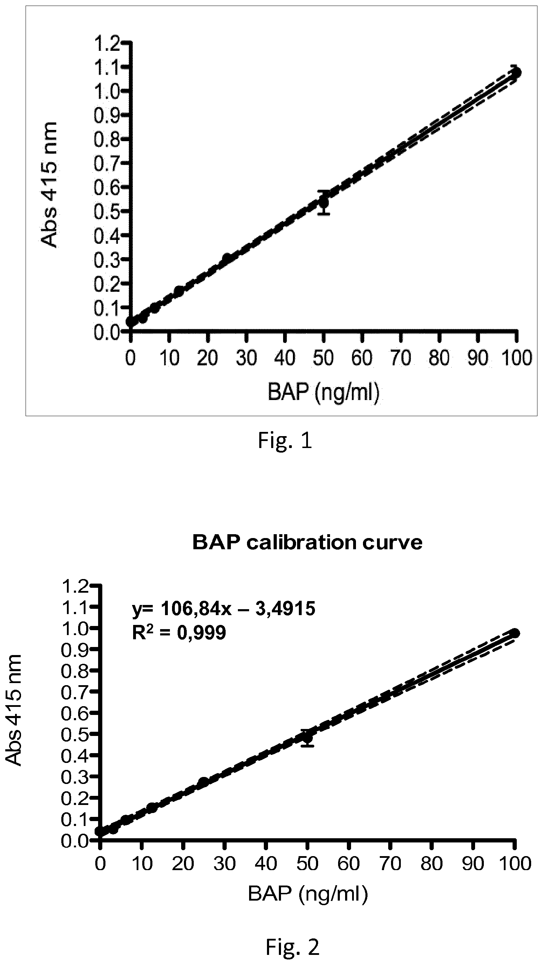

The results of a typical standard calibration curve of the kit with readings at a wavelength of 415 nm are shown in **Table 1** and **Figure 1****.**

**Table 1 -Example Data for Calibration curve construction**

| BAP (ng/ml) | Abs. 415 nm | | | | | Calibrator |
|---|---|---|---|---|---|---|
| | Abs. (1) | Abs. (2) | Abs. (3) | Mean Abs. | Std.Dev. | |
| 100 | 1,086 | 1,026 | 1,119 | 1,077 | 0,047 | **C [6]** |
| 50 | 0,628 | 0,501 | 0,476 | 0,535 | 0,081 | **C [5]** |
| 25 | 0,345 | 0,272 | 0,294 | 0,304 | 0,037 | **C [4]** |
| 12,5 | 0,186 | 0,156 | 0,161 | 0,168 | 0,016 | **C [3]** |
| 6,25 | 0,110 | 0,098 | 0,089 | 0,099 | 0,010 | **C [2]** |
| 3,125 | 0,054 | 0,050 | 0,066 | 0,057 | 0,008 | **C [1]** |
| 0 | 0,045 | 0,041 | 0,040 | 0,042 | 0,002 | **C [0]** |

The calibration curve was constructed by plotting the absorbance obtained for each calibrator on the Y-axis against the BAP concentration in ng/mL for the X-axis. The data analysis was carried out on measurements performed on three different wells of the plate.

GraphPad Prism 5 software was used for the data analysis and graphic construction of the straight line from the interpolation of data; a linear regression curve as shown in **Table 2** was selected for the quantification of BAP levels.

**Table 2. Linear regression analysis: Parameters of calibration curve.**

| **Linear regression line** | **Abs. 415** |
|---|---|
| | |

| **Optimal values** | |
|---|---|
| Slope | 0.0104±0.000127 |
| Y Intercept (X=0.0) | 0.0343±0.00553 |
| X Intercept (Y=0.0) | -3,31 |
| 1/Slope | 96,5 |
| *95% Confidence Interval* | |
| Slope | 0.0100 - 0.107 |
| Y Intercept (X=0.0) | 0.0201 - 0.0485 |
| X Intercept (Y=0.0) | -4.78 - 1.90 |
| *Goodness of prediction* | |
| r² | 0,999 |
| Sy.x | 0,0112 |
| | |
| F | 6690 |
| DFn,DFd | 1.00, 5.00 |
| P-value | < 0.0001 |
| Deviation from zero | Significant |
| *Data* | |
| Number of values X | 7 |
| Maximum Number of replicates Y | 3 |
| Total number of values | 7 |
| Number of values missing or omitted | 0 |

### Performance characteristics

### Sensitivity

The minimum detection limit for BAP levels is 3.125 ng/mL, calculated by interpolation of the mean plus two standard deviations of 20 replicates of the calibrator. The present authors carried out tests with a monoclonal antibody (Abcam- ab17272). The results, however, were unsatisfactory in terms of both sensitivity and specificity, whereas the tests performed with the polyclonal antibody showed shorter and more convenient reaction development times.

### Expected values

In order to evaluate the capability of the new described kit to detect BAP in different situations, the present immunoenzymatic assay (EIA) for analyzing BAP was used to evaluate both human serum samples and the supernatant of SAOS-2 human osteoblastic cells [commercially obtained cell line], which synthesize BAP; the latter can, thus, be detected in the supernatant of cells cultured in vitro.

The values obtained from the analysis of human serum were compared with those obtained by means of a commercially available BAP EIA assay which uses monoclonal antibodies (Ostease®BAP, IDS Ltd., UK). Briefly, the present authors performed the assay evaluating the enzymatic activity both in the serum of patients and in supernatants of osteoblasts. The results are described in Tables 1 and 3. In order to obtain values distributed over a wide assay calibration interval, and also verify the quality of the product, the sensitivity was tested on cellular supernatants from cells pre-conditioned with the serum of obese patients I basasl rest conditions (SAOS-TO) or patients who underwent physical acttivity-based protocols for 4 (SAOS-T4) and 6 months (SAOS-T6), experimental conditions known to positively modify skeletal tissue. Each sample was assayed in duplicate on each plate (100 µicroliters/well).

The values of the calibration curve were used to obtain BAP concentrations (nanograms/ml) corresponding to the read absorbance values.

An example of execution of the assay and the associated analysis of the values obtained is given in **Table 3** and **Figure 2****.**

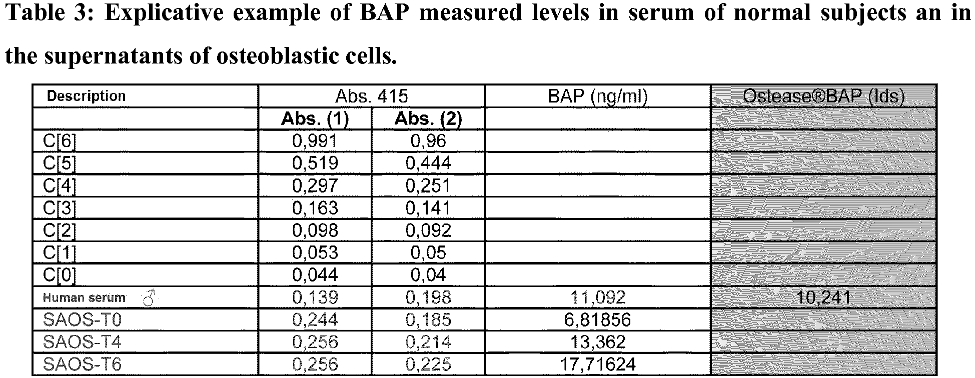

In particular, BAP levels have been measured in the serum of healthy subjects and in the supernatants of osteoblasts exposed to the serum of obese subjects who performed physical activity. In the table are depicted the values detected with the new developed method and a commercialized kit (Ostease).

The results obtained show that the present kit is capable of in vitro evaluation and assaying of human BAP as far as both serum levels and supernatants of osteoblasts, BAP producing cells and the detected values are almost identical to the ones found with the commercialized.

As described, the kit will enable to obtain results in a faster and more economic manner since two different biomarkers will be evaluated in the same diagnostic plate, which will help to reduce the total costs of the assay.

As just mentioned, a distinctive feature of this new diagnostic kit is the potential expansion of the number of markers evaluated with the kit in the same assay, with a further decrease in costs, but it is also a clinical advantage given the obtainment of several markers in the same diagnostic kit.

Figure 2 shows a preliminary OCN analysis performed to develop the additional part to the diagnostic kit.

The ELISA "sandwich" involves the coverage of the bottom of the well with an OCN specific antibody, followed by a secondary antibody introduction, which will bind the antibody + antigen complex, forming a triple layer (or "sandwich"). The last step will involve the addition of an antibody conjugated to ALP and, adding its substrate (pNPP), a colored product will be formed, which will highlight the cockpit (see Materials and Methods).

In a first set of experiments, the specific concentrations and volumes of each antibody used in the "sandwich" were evaluated by using as a standard a synthetic osteocalcin fragment (aa 1-22) in order to obtain an accurate calibration curve. For the preparation of the overcoating of the wells, the OCN specific antibody (Abcam, Ab1857) was diluted in PBS buffer and tested in a dilution range between 0.1-10 micrograms/ml for an incubation period, lasted between 2 and 24 hours. The antibody of "detection" anti-OCN (Abcam, Ab13418) was diluted in PBS and tested in the range of concentration of 1-10 grams/ml, while the secondary antibody conjugated to ALP was diluted in TBS buffer and assayed at two different concentrations: 0.1 and 1 milligrams/ml. The calibration curve was constructed using a fragment of 22 aa of the synthetic peptide of human OCN (Abcam, Ab42234) in the concentration range 2 - 100 nanograms/ml. As shown below, these experiments allowed to identify the concentrations of selected antibodies for the ELISA "sandwich" (see materials and methods) that have permitted to obtain a good linearity of the calibration standard curve for determination of OCN (R² = 0.978) in the concentration range examined.

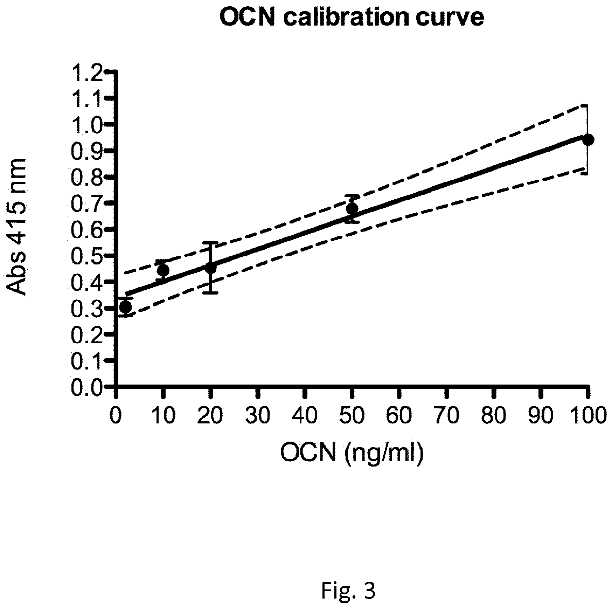

The results of a typical standard calibration curve (wavelength reading of 415 nm) are shown in Figure 3. The calibration curve was developed by plotting the absorbance obtained for each calibrator on the Y-axis, against the OCN concentration in nanograms/mL for the X-axis. The data analysis was performed on measurements carried out on three different wells of the plate. Data analysis and graphic construction was performed selecting a linear regression analysis curve (Table 4).

**Table 4. Analysis of linear regression, which consists in equation which allows to find the best-fitting straight line through the points to allow correlation between two variables. The best-fitting line is called a regression line.**

| | Linear reg. | A |
|---|---|---|
| | | Abs. 415 nm |
| | | |
| 3 | Y-intercept when X=0.0 | 0.3390 ± 0.02693 |
| 4 | X-intercept when Y=0.0 | -54.73 |
| 5 | 1/slope | 161.4 |
| 6 | 95% Confidence Intervals | |
| 7 | Slope | 0.004514 to 0.007874 |
| 8 | Y-intercept when X=0.0 | 0.2533 to 0.4247 |
| 9 | X-intercept when Y=0.0 | -90.49 to -33.45 |
| 10 | Goodness of Fit | |
| 11 | r² | 0.9787 |
| 12 | Sy.x | 0.04217 |
| 13 | Is slope significantly non-zero? | |
| 14 | F | 137.6 |
| 1.5 | DFn, DFd | 1.000, 3.000 |
| 16 | P value | 0.0013 |
| 17 | Deviation from zero? | Significant |
| 18 | Data | |
| 19 | Number of X values | 5 |
| 20 | Maximum number of Y replicates | 1 |
| 21 | Total number of values | 5 |
| 22 | Number of missing values | 0 |
| 23 | Runs test | |
| 24 | Points above line | 2 |

Then, the present invention demonstrates the development of a new faster an economic method and the further possibility to combine detection of BAP and detection of OCN.

### REFERENCES

1. Migliaccio S, Falcone S, Spera G Aging Clinical Experimental Research, 2004;16S:20-2.
2. Adami S, Bertoldo F, Gatti D, Minisola G, Rossini M, Sinigaglia L, Varenna M. Calcif Tissue Int. 2013;93:195-200.
3. NIH Consensus Development Panel on Osteoporosis. Journal of the American Medical Association, 2001, 285:785-795.
4. Patel CG, Yee AJ, Scullen TA, Nemani N, Santo L, Richardson PG, Laubach JP, Ghobrial IM, Schlossman RL, Munshi NC, Anderson KC, Raje NS. Clin Cancer Res. 2014;20(15):3955-61.
5. Greco EA, Lenzi A, Migliaccio S. Horm Mol Biol Clin Investig. 2016 Mar 17.
6. Du WX, Duan SF, Chen JJ, Huang JF, Yin LM, Tong PJ. J Cancer Res Ther. 2014 Nov;10 Suppl:C140-3.
7. Kamiya N, Suzuki H, Endo T, Yano M, Naoi M, Nishimi D, Kawamura K, Imamoto T, Ichikawa T. Int J Urol. 2012;19:968-79.
8. Drweska-Matelska N, Wolski H, Seremak-Mrozikiewicz A, Majchrzycki M, Kujawski R, Czerny B. Ginekol Pol. 2014;85:852-9.
9. Ambroszkiewicz J, Rowicka G, Chelchowska M, Gajewska J, Strucinska M, Laskowska-Klita T. Arch Med Sci. 2014;10(6):1135-41.
10. Borer KT Sports Medicine 2005; 35:779-830.
11. Goseki M, Oida S, Takeda K, Ogata Y, Iimura T, Maruoka Y, Sasaki S. J Dent Res. 1995;74(1):319-22.
12. Romero Barco CM, Manrique Arija S, Rodriguez Pérez M. Reumatol Clin. 2012;8(3):149-52.
13. Weiss MJ, Ray K, Henthorn PS, Lamb B, Kadesch T, Harris H. J Biol Chem. 1988;263(24):12002-10.
14. Saraç F, Saygili F. Biotechnology & Biotechnological Equipment, (2007) 21:2, 194-197.
15. Panigrahi, K, Delmas PD, Singer F, Ryan W, Reiss O, Fisher R, Miller PD, Mizrahi I, Darte C, Kress BC., Christenson RH. Clin. Chem. 40/5, 822-828 (1994).

## Claims

1. An *in vitro* method for the diagnosis of a skeletal disorder in a biological sample of a subject comprising the following steps:
a) contact and incubation of the biological sample with an anti-human bone alkaline phosphatase (BAP) polyclonal antibody thereby forming a BAP-antibody complex, if BAP is present;
b) separation of the biological sample from the BAP-antibody complex;
c) selective detection of BAP bound to the polyclonal antibody and/or quantifying the amount of BAP bound to the polyclonal antibody using detecting means for the antibody;
d) comparison of the result obtained in c) to a control result, preferably wherein the anti-human bone alkaline phosphatase (BAP) polyclonal antibody is immobilized to a solid support wherein the biological sample is selected from the group consisting of: serum, plasma, saliva , urine, a supernatant of osteoblast cells, preferably wherein the osteoblast cells are *in vitro* cultivated and/or stimulated with subject's serum and
wherein an amount of BAP greater than the amount of the control sample indicates that the subject is affected by a skeletal disorder selected from the group consisting of: osteoporosis, osteosarcoma, Paget's disease, osteodystrophy, osteopetrosis,
or wherein an amount of BAP lower than the amount of the control sample indicates that the subject is affected by a skeletal disturb is hypophosphatasia.

2. The method according to claim 1 wherein an amount of BAP greater or lower than the amount of the control sample indicates that the subject is affected by a skeletal disorder or is at risk of developing a skeletal disorder.

3. The method according to any one of the previous claims, wherein the anti-human BAP polyclonal antibody is an IgG.

4. The method according to any one of the previous claims, wherein the anti-human BAP polyclonal antibody is produced in sheep.

5. The method according to any one of previous claim, wherein the amount of a further skeletal disorder marker is detected and/or measured.

6. The method according to claim 5, wherein the further skeletal disorder marker is osteocalcin.

7. Use of a kit comprising an anti-BAP polyclonal antibody for carrying out the method of claims 1-6.

8. Use of the kit according to claim 7 wherein the kit further comprises means to detect and/or quantify a BAP-antibody complex.

9. Use of the kit according to claim 7 or 8, wherein the method is an immunoassay.

10. Use of the kit according to any one of claims 7-9, wherein the method detects and/or quantifies the human bone alkaline phosphatase (BAP) in the biological sample.

11. Use of the kit according to any one of claim 7 to 10 further comprising a solid support wherein the antibody is immobilized.

12. Use of the kit according to claim 11 wherein the solid support is a microtitration plate.

13. Use of the kit according to any one of claims 7-12, wherein the anti-BAP polyclonal antibody is an IgG.

14. Use of the kit according to any one of claims 7-13, wherein the anti-human BAP polyclonal antibody is produced in sheep.

## Patentansprüche

1. In-vitro-Verfahren zur Diagnose einer Skeletterkrankung in einer biologischen Probe eines Patienten, die folgenden Schritte umfassend:
a) Kontakt und Inkubation der biologischen Probe mit einem polyklonalen Antikörper gegen humane alkalische Knochenphosphatase (BAP), wodurch ein BAP-Antikörper-Komplex gebildet wird, falls BAP vorhanden ist;
b) Trennung der biologischen Probe vom BAP-Antikörper-Komplex;
c) selektiver Nachweis des an den polyklonalen Antikörper gebundenen BAP und/oder Quantifizierung der Menge des an den polyklonalen Antikörper gebundenen BAP unter Verwendung von Nachweismitteln für den Antikörper;
d) Vergleich des in c) erhaltenen Ergebnisses mit einem Kontrollergebnis, bei dem vorzugsweise der polyklonale Antikörper gegen menschliche alkalische Knochenphosphatase (BAP) auf einem festen Träger immobilisiert ist,
wobei die biologische Probe aus der Gruppe ausgewählt wird, die besteht aus: Serum, Plasma, Speichel, Urin, einem Überstand von Osteoblastenzellen, vorzugsweise wobei die Osteoblastenzellen *in vitro* mit dem Serum des Patienten kultiviert und/oder stimuliert werden, und
wobei eine BAP-Menge, die größer als die Menge der Kontrollprobe ist, anzeigt, dass der Patient von einer Skeletterkrankung betroffen ist, die aus der Gruppe ausgewählt wird, die aus Osteoporose, Osteosarkom, Paget-Krankheit, Osteodystrophie, Osteopetrose besteht,
oder wobei eine BAP-Menge, die geringer ist als die Menge der Kontrollprobe, darauf hinweist, dass die Person von einer Skelettstörung betroffen ist, die Hypophosphatasie ist.

2. Verfahren nach Anspruch 1, bei dem eine BAP-Menge, die größer oder kleiner als die Menge der Kontrollprobe ist, anzeigt, dass der Patient von einer Skeletterkrankung betroffen ist oder das Risiko hat, eine Skeletterkrankung zu entwickeln.

3. Verfahren nach einem der vorhergehenden Ansprüche, wobei der polyklonale anti-humane BAP-Antikörper ein IgG ist.

4. Verfahren nach einem der vorstehenden Ansprüche, bei dem der polyklonale anti-humane BAP-Antikörper in Schafen hergestellt wird.

5. Verfahren nach einem der vorherigen Ansprüche, bei dem die Menge eines zusätzlichen Markers für Skelettstörungen nachgewiesen und/oder gemessen wird.

6. Verfahren nach Anspruch 5, wobei der weitere Marker für eine Skeletterkrankung Osteocalcin ist.

7. Verwendung eines Kits, das einen polyklonalen Anti-BAP-Antikörper enthält, zur Durchführung des Verfahrens nach den Ansprüchen 1-6.

8. Verwendung des Kits nach Anspruch 7, wobei das Testkit weiterhin Mittel zum Nachweis und/oder zur Quantifizierung eines BAP-Antikörper-Komplexes umfasst.

9. Verwendung des Kits nach Anspruch 7 oder 8, wobei das Verfahren ein Immunoassay ist.

10. Verwendung des Kits nach einem der Ansprüche 7-9, wobei das Verfahren die menschliche alkalische Knochenphosphatase (BAP) in der biologischen Probe nachweist und/oder quantifiziert.

11. Verwendung des Kits nach einem der Ansprüche 7 bis 10, ferner einen festen Träger umfassend, worin der Antikörper immobilisiert ist.

12. Verwendung des Kits nach Anspruch 11, wobei der feste Träger eine Mikrotitrationsplatte ist.

13. Verwendung des Kits nach einem der Ansprüche 7-12, wobei der polyklonale Anti-BAP-Antikörper ein IgG ist.

14. Verwendung des Kits nach einem der Ansprüche 7-13, wobei der polyklonale anti-humane BAP-Antikörper in Schafen hergestellt wird.

## Revendications

1. Procédé in vitro destiné à diagnostiquer une maladie squelettique dans un échantillon biologique d'un patient, comprenant les étapes suivantes :
a) contact et incubation de l'échantillon biologique avec un anticorps polyclonal anti phosphatase alcaline osseuse (PAO) humaine, formant ainsi un complexe d'anticorps PAO, si une PAO est présente ;
b) séparation de l'échantillon biologique d'avec le complexe d'anticorps PAO ;
c) détection sélective de PAO liée à l'anticorps polyclonal et/ou quantification de la quantité de PAO liée à l'anticorps polyclonal à l'aide d'un moyen de détection pour l'anticorps ;
d) comparaison du résultat obtenu à l'étape c) avec un résultat de contrôle, l'anticorps polyclonal anti phosphatase alcaline osseuse (PAO) humaine étant de préférence immobilisé sur un support solide, l'échantillon biologique étant sélectionné parmi le groupe comprenant : le sérum, le plasma, la salive, l'urine, un surnageant de cellules ostéoblastes, les cellules ostéoblastes étant de préférence cultivées in vitro et/ou stimulées avec du sérum du patient et
dans lequel une quantité de PAO supérieure à la quantité de l'échantillon de contrôle indique que le patient est atteint d'une maladie squelettique sélectionnée parmi le groupe comprenant : l'ostéoporose, l'ostéosarcome, la maladie de Paget, l'ostéodystrophie, l'ostéopétrose,
ou dans lequel une quantité de PAO inférieure à la quantité de l'échantillon de contrôle indique que le patient est atteint d'un trouble squelettique appelé hypophosphatasie.

2. Procédé selon la revendication 1, dans lequel une quantité de PAO supérieure ou inférieure à la quantité de l'échantillon de contrôle indique que le patient est atteint d'une maladie squelettique ou risque de développer une maladie squelettique.

3. Procédé selon l'une quelconque des revendications précédentes, dans lequel l'anticorps polyclonal anti PAO humaine est un IgG.

4. Procédé selon l'une quelconque des revendications précédentes, dans lequel l'anticorps polyclonal anti PAO humaine est produit dans le mouton.

5. Procédé selon l'une quelconque des revendications précédentes, dans lequel la quantité d'un autre marqueur de maladie squelettique est détectée et/ou mesurée.

6. Procédé selon la revendication 5, dans lequel l'autre marqueur de maladie squelettique est l'ostéocalcine.

7. Utilisation d'un kit comprenant un anticorps polyclonal anti PAO, pour la mise en œuvre du procédé selon les revendications 1 à 6.

8. Utilisation du kit selon la revendication 7, dans laquelle le kit comprend en outre un moyen destiné à détecter et/ou à quantifier un complexe d'anticorps PAO.

9. Utilisation du kit selon la revendication 7 ou 8, dans laquelle le procédé est un dosage immunologique.

10. Utilisation du kit selon l'une quelconque des revendications 7 à 9, dans laquelle le procédé détecte et/ou quantifie la phosphatase alcaline osseuse (PAO) humaine dans l'échantillon biologique.

11. Utilisation du kit selon l'une quelconque des revendications 7 à 10, comprenant en outre un support solide dans lequel l'anticorps est immobilisé.

12. Utilisation du kit selon la revendication 11, dans laquelle le support solide est une plaque de micro-titrage.

13. Utilisation du kit selon l'une quelconque des revendications 7 à 12, dans laquelle l'anticorps polyclonal anti PAO est un IgG.

14. Utilisation du kit selon l'une quelconque des revendications 7 à 13, dans laquelle l'anticorps polyclonal anti PAO est produit dans le mouton.


### SEQUENCE LISTING

<110> Università degli Studi di Roma "La Sapienza"
<120> IN VITRO METHOD FOR A NEW DIAGNOSTIC KIT FOR DIAGNOSIS OF SKELETAL DISORDERS
<130> PCT130342
<150> 102015000053012
   <151> 2015-09-18
<160> 3
<170> PatentIn version 3.5
<210> 1
   <211> 342
   <212> DNA
   <213> Homo sapiens
<220>
   <221> misc_feature
   <222> (115)..(214)
   <223> n is a, c, g, or t
<400> 1
   cgcgcccgct atcctggctc cgtgctccca cgcgcttgtg cctggacgga ccctcgccag      60
   tgctctgcgc aggattggaa catcagttaa catctgacca ctgccagccc acccnnnnnn     120
   nnnnnnnnnn nnnnnnnnnn nnnnnnnnnn nnnnnnnnnn nnnnnnnnnn nnnnnnnnnn     180
   nnnnnnnnnn nnnnnnnnnn nnnnnnnnnn nnnngaacac acacgccagc tcctctctga     240
   agcgactctc ctgtttggaa cggcaaaaaa aaattttttt ttctcttttt ggtggtggtt     300
   aaaagggaac acaaaacatt taaataaaac tttccaaata ca                        342
<210> 2
   <211> 29
   <212> PRT
   <213> Homo sapiens
<400> 2
   Arg Ala Arg Tyr Pro Gly Ser Val Leu Pro Arg Ala Cys Ala Trp Thr
   1               5                   10                  15
   Asp Pro Arg Gln Cys Ser Ala Gln Asp Trp Asn Ile Ser
               20                  25
<210> 3
   <211> 524
   <212> PRT
   <213> Homo sapiens
<400> 3
   Met Ile Ser Pro Phe Leu Val Leu Ala Ile Gly Thr Cys Leu Thr Asn
   1               5                   10                  15
   Ser Leu Val Pro Glu Lys Glu Lys Asp Pro Lys Tyr Trp Arg Asp Gln
               20                  25                  30
   Ala Gln Glu Thr Leu Lys Tyr Ala Leu Glu Leu Gln Lys Leu Asn Thr
           35                  40                  45
   Asn Val Ala Lys Asn Val Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Pro Gly Glu Glu Thr Arg Leu Glu Met Asp Lys Phe Pro Phe Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Glu Arg Ser Arg Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met His Asn Ile Arg Asp Ile Asp Val Ile Met Gly Gly Gly Arg Lys
       210                 215                 220
   Tyr Met Tyr Pro Lys Asn Lys Thr Asp Val Glu Tyr Glu Ser Asp Glu
   225                 230                 235                 240
   Lys Ala Arg Gly Thr Arg Leu Asp Gly Leu Asp Leu Val Asp Thr Trp
                   245                 250                 255
   Lys Ser Phe Lys Pro Arg Tyr Lys His Ser His Phe Ile Trp Asn Arg
               260                 265                 270
   Thr Glu Leu Leu Thr Leu Asp Pro His Asn Val Asp Tyr Leu Leu Gly
           275                 280                 285
   Leu Phe Glu Pro Gly Asp Met Gln Tyr Glu Leu Asn Arg Asn Asn Val
       290                 295                 300
   Thr Asp Pro Ser Leu Ser Glu Met Val Val Val Ala Ile Gln Ile Leu
   305                 310                 315                 320
   Arg Lys Asn Pro Lys Gly Phe Phe Leu Leu Val Glu Gly Gly Arg Ile
                   325                 330                 335
   Asp His Gly His His Glu Gly Lys Ala Lys Gln Ala Leu His Glu Ala
               340                 345                 350
   Val Glu Met Asp Arg Ala Ile Gly Gln Ala Gly Ser Leu Thr Ser Ser
           355                 360                 365
   Glu Asp Thr Leu Thr Val Val Thr Ala Asp His Ser His Val Phe Thr
       370                 375                 380
   Phe Gly Gly Tyr Thr Pro Arg Gly Asn Ser Ile Phe Gly Leu Ala Pro
   385                 390                 395                 400
   Met Leu Ser Asp Thr Asp Lys Lys Pro Phe Thr Ala Ile Leu Tyr Gly
                   405                 410                 415
   Asn Gly Pro Gly Tyr Lys Val Val Gly Gly Glu Arg Glu Asn Val Ser
               420                 425                 430
   Met Val Asp Tyr Ala His Asn Asn Tyr Gln Ala Gln Ser Ala Val Pro
           435                 440                 445
   Leu Arg His Glu Thr His Gly Gly Glu Asp Val Ala Val Phe Ser Lys
       450                 455                 460
   Gly Pro Met Ala His Leu Leu His Gly Val His Glu Gln Asn Tyr Val
   465                 470                 475                 480
   Pro His Val Met Ala Tyr Ala Ala Cys Ile Gly Ala Asn Leu Gly His
                   485                 490                 495
   Cys Ala Pro Ala Ser Ser Ala Gly Ser Leu Ala Ala Gly Pro Leu Leu
               500                 505                 510
   Leu Ala Leu Ala Leu Tyr Pro Leu Ser Val Leu Phe
           515                 520